# EUROPÄISCHE PATENTANMELDUNG

(11) **EP 1 400 505 A1**
(43) Veröffentlichungstag der Anmeldung: **24.03.2004**
(21) Anmeldenummer: 03021071.0
(22) Anmeldetag: 18.09.2003
(51) Int. Cl.: C07C 45/68, C07D 315/00

(54) **Verfahren zur Herstellung von makrocyclischen Ketonen und Lactonen**

(30) Priorität: 20.09.2002 DE 10243668
(71) Anmelder: Symrise GmbH & Co. KG, 37603 Holzminden (DE)
(72) Erfinder: Eh, Marcus, 37603 Holzminden (DE); Surburg, Horst, 37603 Holzminden (DE)
(74) Vertreter: Eisenführ, Speiser & Partner

(57) **Zusammenfassung**

Die Erfindung betrifft ein neues Verfahren zur Herstellung von ungesättigten, methylsubstituierten makrocyclischen Ketonen und Lactonen der Formel (I) 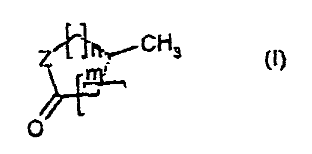 worin Z = -CH₂-oder-O-ist, m und n die in der Beschreibung angegebenen Bedeutungen haben, und wobei die unterbrochenen Linien eine C-C-Einfach- und die andere eine C=C-Doppelbindung bedeuten,
das dadurch gekennzeichnet ist, dass als Edukt ein Ketolacton oder Diketon der Formel (II) 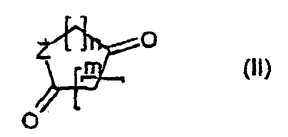 worin
Z, n und m die oben genannte Bedeutung haben,
in einer Grignard Reaktion mit Methylmagnesiumhalogenid und anschließender saurer Dehydratisierung zu den ungesättigten, methylsubstituierten makrocyclischen Ketonen und Lactonen der Formel (I) umgesetzt wird.

## Beschreibung

Die Erfindung betrifft ein neues Verfahren zur Herstellung von ungesättigten, methylsubstituierten makrocyclischen Ketonen und Lactonen.

Verbindungen mit Moschusgeruch sind begehrte Komponenten in der Duftstoffindustrie. Sie zeichnen sich sowohl durch ihre Eigenschaft Parfümkompositionen Ausstrahlung zu verleihen, als auch durch ihre Fähigkeit als Fixateur zu wirken, aus. Somit kommen heutzutage Moschusriechstoffe in vielen Parfümkompositionen zum Einsatz.

Die Klasse der naturähnlichen makrocyclischen Moschusriechstoffe wird in Zukunft immer mehr an Bedeutung gewinnen, da die synthetischen Moschusverbindungen der nitroaromatischen und polycyclischen Reihe persistent und lipophil sind, so dass diese Verbindungen sich in aquatischen Nahrungsketten und Fettgewebe anreichern (Emährungs-Umschau 1996, 43, 442-449; Emährungs-Umschau 1997, 44, 4-9).

Typische Moschusriechstoffe zeichnen sich durch einen makrocyclischen Ring mit 13 bis 17 C-Atomen aus, welcher als funktionelle Gruppe ein Keton oder einen Ester trägt. Weiterhin ist eine Alkylsubstitution, hierbei bevorzugt eine Methylsubstitution, im makrocyclischen Ring möglich.

Methylsubstituierte ungesättigte makrocyclische Lactone können aus den entsprechenden Ketolactonen durch Methylenierung via Wittig Reaktion und anschließender saurer Doppelbindungsisomerisierung hergestellt werden (DE-A 19 801 056). Methylsubstituierte ungesättigte makrocyclische Ketone können aus den entsprechenden Diketonen entweder durch Methylenierung via Wittig Reaktion und anschließender saurer Doppelbindungsisomerisierung hergestellt werden (DE-A 10 038 021) oder durch Umsetzung des Diketons mit 2 Äquivalenten Natriumhydrid und drei Äquivalenten Methyllithium und anschließender saurer Dehydratisierung (Monatsh., 1979,110, 245-247).

Die vorgenannten Verfahren zur Herstellung von methylsubstituierten, ungesättigten makrocyclischen Ketonen und Lactonen bedienen sich einerseits der teuren und im technischen Maßstab relativ schwer durchführbaren Wittig Reaktion oder andererseits der Umsetzung mit einem großen Überschuss an Methyllithium. Die Aufgabe der vorliegenden Erfindung bestand darin ein Alternativverfahren zu entwickeln, wobei aus den entsprechenden Ketolactonen oder Diketonen die geruchlich interessanten ungesättigten, methylsubstituierten makrocyclischen Ketone und Lactone gebildet werden.

Es wurde ein Alternativverfahren zur Herstellung von ungesättigten, methylsubstituierten makrocyclischen Ketonen und Lactonen der Formel (I) 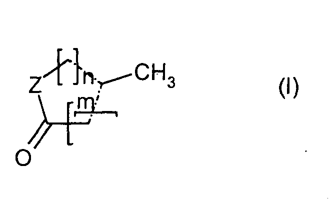 gefunden,
worin
Z = -CH₂- ist,
n, m ≥ 1 und n + m = 11 bis 15, bevorzugt 12 bis 14 sind, besonders bevorzugt sind n = 2, 3, 5 oder 6 und n + m = 12 bis 14,
oder
Z = 0 ist,
n ≥ 0, m ≥ 1 und n + m = 11 bis 15, bevorzugt 12 bis 14 sind, besonders bevorzugt sind n = 2, 3 oder 4 und n + m = 12 bis 14,
wobei die unterbrochenen Linien eine C-C-Einfach- und die andere eine C=C-Doppelbindung bedeuten,
das dadurch gekennzeichnet ist, dass als Edukt ein Ketolacton oder Diketon der Formel (II) 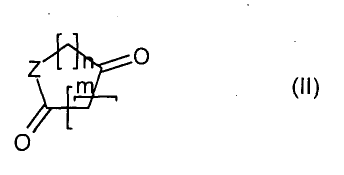 worin
Z, n und m die oben genannte Bedeutung haben,
in einer Grignard Reaktion mit Methylmagnesiumhalogenid und anschließender saurer Dehydratisierung zu den ungesättigten, methylsubstituierten makrocyclischen Ketonen und Lactonen der Formel (I) umgesetzt wird.

Im ersten Schritt werden typischerweise 0,5 bis 2,0 Äquivalente, bevorzugt 0,8 bis 1,5 Äquivalente, MeMgX mit X = Halogen, bevorzugt Chlorid, bezogen auf (II) eingesetzt.

Die Konzentration der MeMgX-Lösung ist typischerweise 1,0 bis 3,0 molar, in einem aprotischen Lösungsmittel, bevorzugt Diethylether, Tetrahydrofuran, 1,2-Dimethoyethan, 1,2-Diethoxyethan, Hexan, Heptan, Toluol, besonders bevorzugt Tetrahydrofuran 1,2-Dimethoyethan, 1,2-Diethoxyethan, und Toluol und Mischungen davon.

Vorteilhafterweise gibt man die MeMgX-Lösung zu einer 0,5 bis 3,5 molaren; bevorzugt 1,0 bis 3,0 molaren Lösung, bestehend aus dem Molekül der Formel (II) und einem aprotischen Lösungsmittel, bevorzugt Diethylether, Tetrahydrofuran, 1,2-Dimethoyethan, 1,2-Diethoxyethan, Hexan, Heptan, Toluol, besonders bevorzugt Tetrahydrofuran 1,2-Dimethoyethan, 1,2-Diethoxyethan und Toluol und Mischungen davon.

Die Zugabe der MeMgX-Lösung zu der Diketon-Lösung (Z = -CH₂-) vorteilhafterweise bei einer Temperatur von 25°C bis 150°C, bevorzugt 35°C bis 100°C, besonders bevorzugt 40°C bis 90°C. Nach beendeter Zugabe der MeMgX-Lösung lässt man die resultierende Reaktionslösung bei einer Temperatur von 25°C bis 150°C, bevorzugt 35°C bis 100°C, besonders bevorzugt 40°C bis 90°C über einen Zeitraum von 30 Minuten bis 10 Stunden, bevorzugt 1 Stunde bis 5 Stunden nachrühren.

Als Zwischenprodukt aus der ersten Stufe für den Fall Z = -CH₂- bildet sich ein Gemisch aus dem Diketon der Formel (III), dem Hydroxyketon der Formel (IV) und dem Diol der Formel (V). 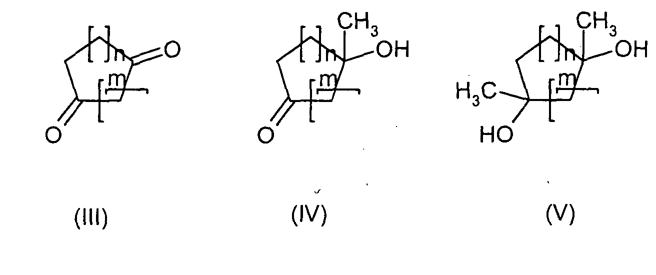 worin,
n, m ≥ 1 und n + m = 11 bis 15, bevorzugt 12 bis 14 sind, besonders bevorzugt sind n = 2, 3, 5 oder 6 und n + m = 12 bis 14.

Die Zwischenprodukte der Formel (IV), wobei n und m die oben genannte Bedeutung haben, sind neu, mit der Ausnahme n = 1 und m = 11. Der GC-Gehalt (Gehalt im Gaschromatogramm) der gewünschten Zwischenprodukte der Formel (IV), wobei n und m die oben genannte Bedeutung haben, liegt im Bereich von 30 bis 70 %.

Das Reaktionsgemisch aus den drei oben beschriebenen Verbindungen kann entweder aufgereinigt (Destillation, Chromatographie) werden, so dass die Verbindungen der Formel (III), wobei n und m die oben genannte Bedeutung haben, als Reinstoffe in die Folgereaktion eingesetzt werden, oder das Reaktionsgemisch wird ohne weitere Reinigung in die Folgereaktion eingesetzt.

Die Zugabe der MeMgX-Lösung zu der Ketolacton-Lösung (Z = O) vorteilhafterweise bei einer Temperatur von -25°C bis +25°C, bevorzugt -15°C bis +10°C. Nach beendeter Zugabe der MeMgX-Lösung lässt man die resultierende Reaktionslösung bei einer Temperatur von -25°C bis +25°C, bevorzugt -15°C bis +10°C über einen Zeitraum von 30 Minuten bis 10 Stunden, bevorzugt 1 Stunde bis 5 Stunden nachrühren.

Die sich bildenden Zwischenprodukte für den Fall Z = O der Formel (VI) 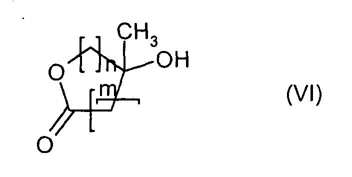 worin, n ≥ 0, m ≥ 1 und n + m = 11 bis 15, bevorzugt 12 bis 14 sind, besonders bevorzugt sind n = 2, 3 oder 4 und n + m = 12 bis 14,
sind neu.

In einem zweiten Schritt werden die Hydroxyketone der Formel (IV) oder die Hydroxylactone der Formel (VI) nach dem Fachmann wohlvertrauten Methoden ((a) H.G.O. Becker et al, Organikum, Johann Ambrosium Verlag, 20. Auflage, 1996, 251 ff; (b) L.F. Tietze, Th. Eicher, Reaktionen und Synthesen, Georg Thieme Verlag, 2. Auflage, 1991, 35) unter sauren Bedingungen dehydratisiert, so dass sich die ungesättigten, methylsubstituierten makrocyclischen Ketone oder Lactone der Formel (I) bilden.

Die folgenden Verbindungen zeigen eine besonders vorteilhafte moschuskörnerhafte Note und sind bisher in der Literatur nicht beschrieben:
5-Methyl-(E/Z)-4/(E/Z)-5-cyclopentadecenon
4-Methyl-(E/Z)-3/(E/Z)-4-cyclohexadecenon
6-Methyl-(E/Z)-5/(E/Z)-6-cyclohexadecenon
3-Methyl-(E/Z)-2/(E/Z)-3-cycloheptadecenon
9-Methyl-(E/Z)-8/(E/Z)-9-cycloheptadecenon
4-Methyloxacyclopentadec-(E/Z)-3/(E/Z)-4-en-2-on
5-Methyloxacyclopentadec-(E/Z)-4/(E/Z)-5-en-2-on
6-Methyloxacyclopentadec-(E/Z)-5/(E/Z)-6-en-2-on
13-Methyloxacyclopentadec-(E/Z)-12/(E/Z)-13-en-2-on
4-Methyloxacyclohexadec-(E/Z)-3/(E/Z)-4-en-2-on
5-Methyloxacyclohexadec-(E/Z)-4/(E/Z)-5-en-2-on
13-Methyloxacyclohexadec-(E/Z)-12/(E/Z)-13-en-2-on
5-Methyloxacycloheptadec-(E/Z)-4/(E/Z)-5-en-2-on
13-Methyloxacycloheptadec-(E/Z)-12/(E/Z)-13-en-2-on

Hiervon zeigen im besonderen Maße folgende Verbindungen eine ausgeprägte moschuskörnerhafte Note:
5-Methyl-(E/Z)-4/(E/Z)-5-cyclopentadecenon
6-Methyl-(E/Z)-5/(E/Z)-6-cyclohexadecenon
13-Methyloxacyclopentadec-(E/Z)-12/(E/Z)-13-en-2-on
13-Methyloxacyclohexadec-(E/Z)-12/(E/Z)-13-en-2-on
13-Methyloxacycloheptadec-(E/Z)-12/(E/Z)-13-en-2-on

Die erfindungsgemäßen Verbindungen 5-Methyl-(E/Z)-4/(E/Z)-5-cyclopentadecenon, 4-Methyl-(E/Z)-3/(E/Z)-4-cyclohexadecenon, 6-Methyl-(E/Z)-5/(E/Z)-6-cyclohexadecenon, 3-Methyl-(E/Z)-2/(E/Z)-3-cycloheptadecenon, 9-Methyl-(E/Z)-8/(E/Z)-9-cycloheptadecenon, 4-Methyloxacyclopentadec-(E/Z)-3/(E/Z)-4-en-2-on, 5-Methyloxacyclopentadec-(E/Z)-4/(E/Z)-5-en-2-on, 6-Methyloxacyclopentadec-(E/Z)-5/(E/Z)-6-en-2-on, 13-Methyloxacyclopentadec-(E/Z)-12/(E/Z)-13-en-2-on, 4-Methyloxacyclohexadec-(E/Z)-3/(E/Z)-4-en-2-on, 5-Methyloxacyclohexadec-(E/Z)-4/(E/Z)-5-en-2-on, 13-Methyloxacyclohexadec-(E/Z)-12/(E/Z)-13-en-2-on, 5-Methyloxacycloheptadec-(E/Z)-4/(E/Z)-5-en-2-on, 13-Methyloxacycloheptadec-(E/Z)-12/(E/Z)-13-en-2-on können insbesondere als Riechstoffe, in Parfümkompositionen, Parfümölen oder Duftkompositionen verwendet werden.

Die erfindungsgemäßen Verbindungen zeichnen sich durch ein hohes Aufziehvermögen und eine hohe Substantivität aus. Dieser Effekt zeigt sich insbesondere auf Haut, Haar und textilen Fasern (z.B. Wolle, Baumwolle, Leinen, synthetische Fasern).

Neben ihren fixierenden Eigenschaften, die Haftfestigkeit von Riechstoffmischungen und Parfümkompositionen zu verbessern (also als Fixateure wirken) erhöhen die Verbindungen die geruchlichen Wahrnehmung, d.h. sie sind sogenannte Enhancer oder Booster.

Besonders geeignete Verwendungsgebiete sind daher Waschmittel, Hygiene- oder Pflegeprodukte, insbesondere im Bereich der Körperpflege, der Kosmetik und des Haushalts.

Die erfindungsgemäßen Verbindungen enthaltenden Parfümöle können in konzentrierter Form, in Lösungen oder in sonstiger modifizierter Form verwendet werden für die Herstellung von z.B. Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und -lotionen, Gesichtscremes und -lotionen, Sonnenschutzcremes- und -lotionen, After-sun-cremes und -lotionen, Handcremes und - lotionen, Fußcremes und -lotionen, Enthaarungscremes und -lotionen, After-shave-Cremes und -lotionen, Bräunungscremes und -lotionen, Haarpflegeprodukten wie z.B. Haarsprays, Haargelen, Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Roll-ons, Deosticks, Deocremes oder Produkten der dekorativen Kosmetik.

Die folgenden Beispiele erläutern die Erfindung:

### Beispiele:

### Beispiel 1:

### (8/9)-Methyl-(E/Z)-7/(E/Z)-8-cyclohexadecenon

*(8*/*9)-Hydroxy-(8*/*9)-methyl-cyclohexadecanon:* Man legt 256,8 g (1,0 Mol) (1,8/1,9)-Cyclohexadecandion in 1000 ml Toluol vor und erhitzt auf 70°C. Jetzt tropft man 833 ml 1,5 molare (1.25 Mol) MeMgCl-Lösung in THF/Toluol =1:1 innerhalb von 6 Stunden zu. Nach beendetem Zutropfen rührt man noch 60 Minuten bei 70°C, bevor man die Reaktionslösung auf Raumtemperatur abkühlen lässt. Jetzt gibt man unter Rühren die abgekühlte Reaktionslösung zu 450 g 20 %iger Essigsäurelösung, rührt weitere 15 Minuten, trennt anschließend die Phasen, trocknet die organische Phase über Na₂SO₄ filtriert ab und entfernt das Lösungsmittel am Rotationsverdampfer. Man erhält 246 g Rohprodukt, welches einen GC-Gehalt an (8/9)-Hydroxy-(8/9)-methyl-cyclohexadecanon von 41,4 % aufweist. Das Rohprodukt wird ohne weitere Reinigung in die Dehydratisierung eingesetzt. Zur Absicherung der Struktur von (8/9)-Hydroxy-(8/9)-methyl-cyclohexadecanon wurde eine kleine Probe des Rohproduktes säulenchromatographisch (Cyclohexan/EtOAc = 10:1) gereinigt, so dass die Struktur von (8/9)-Hydroxy-(8/9)-methyl-cyclohexadecanon bestätigt werden konnte.
¹H-NMR (200 MHz, CDCl₃): δ (ppm) = 1,16 (s, 3H), 1,17-1,52 (m, 21H), 1,58-1,72 (4H), 2,39-2,44 (m, 4H).
¹³C-NMR (50MHz, CDl₃): δ (ppm) = 22,5, 22,6, 22,8, 24,2, 27,3, 27,4, 27,7, 27,8, 27,9, 28,0, 29,0, 39,0, 39,7, 41,1, 42,4, 73,1, 212,5.

*(8*/*9)-Methyl-(E*/*Z)-7*/*(E*/*Z)-8-cyclohexadecenon:* Man legt 240 g Rohprodukt der 1. Stufe vor, gibt 4.8 g (2 Gew.%) H₂SO₄ konz. hinzu und erhitzt für 1 Stunde auf 80°C. Jetzt lässt man abkühlen und gibt anschließend 50.0 g gesättigte NaHCO₃-Lösung hinzu, rührt 15 Minuten und trennt anschließend die Phasen. Man erhält 194 g Rohprodukt mit einem GC-Gehalt an (8/9)-Methyl-(E/Z)-7/(E/Z)-8-cyclohexadecenon von 43%. Nach Destillation an einer 20-bödigen Kolonne erhält man (8/9)-Methyl-(E/Z)-7/(E/Z)-8-cyclohexadecenon in einer Menge von 69 g (Sdp.: 113-115°C, 0,6 mbar) und einer GC-Reinheit (Reinheit im Gaschromatogramm) von 99,2 %.

### Beispiel 2:

### 13-Methyloxacyclohexadec-(E/Z)-12/(E/Z)-13-en-2-on

*13-Hydroxy-13-methyl-oxacyclohexadecan-2-on:* Man legt 12,7 g (50 mmol) Oxacyclohexadecan-2,13-dion in 150 ml THF vor und kühlt auf -15°C ab. Anschließend tropft man 25 ml einer 3,0 molaren MeMgCl-Lösung in THF innerhalb von 30 Minuten zu, so dass die Temperatur von 0°C nicht überstiegen wird. Man rührt weitere 5 Stunden bei -15°C nach, bevor man die Reaktionslösung auf 100 ml kalte, gesättigte NH₄Cl-Lösung gießt, die Phasen trennt und die wässrige Phase noch dreimal mit Dichlormethan extrahiert. Die organischen Phasen werden vereinigt, über Na₂SO₄ getrocknet, abfiltriert und am Rotationsverdampfer von Lösungsmittel befreit. Man erhält 13,5 g Rohprodukt mit einem GC-Gehalt an 13-Hydroxy-13-methyl-oxacyclohexadecan-2-on von 89 %. Das Rohprodukt wird ohne weitere Reinigung in die folgende Dehydratisierung eingesetzt.
¹H-NMR (200 MHz, CDCl₃): δ (ppm) = 1.20 (s, 3H), 1.22-1.42 (m, 20H), 1.52-1.72 (m, 2H), 2.33 (dd, J = 6.6, 5.3 Hz, 2H), 4.14 (q, J = 5.0 Hz, 2H).
¹³C-NMR (50MHz, CDl₃): δ (ppm) = 21,8, 23,7, 24,7, 25,1, 26,2, 26,3, 26,8, 27,3, 27,4, 28,2, 34,7, 36,5, 40,5, 64,3, 72,6, 173,8.

*13-Methyloxacyclohexadec-(E*/*Z)-12*/*(E*/*Z)-13-en-2-on:* Man legt 13,5 g Rohprodukt in 50 ml Toluol vor und fügt 171 mg (0,89 mmol) p-Toluolsulfonsäure Monohydrat hinzu. Jetzt erhitzt man am Wasserabscheider bis sich keine sichtbaren Mengen Wasser mehr abscheiden. Anschließend lässt man die Reaktionslösung auf Raumtemperatur abkühlen, wäscht die organische Phase einmal mit gesättigter NaHCO₃-Lösung, trocknet die organische Phase über Na₂SO₄, filtriert ab und befreit die organische Phase am Rotationsverdampfer von Lösungsmittel. Man erhält 12,5 g Rohprodukt mit einem Gehalt an 13-Methyloxacyclohexadec-(E/Z)-12/(E/Z)-13-en-2-on von 88 %. Nach flashchromatographischer Reinigung (Cyclohexan/EtOAc = 20:1, R_{f}= 0.23) erhält man 9,7 g eines farblosen Öls mit einer Reinheit von 99,4 %.
Geruch: Moschus, erogen, sehr schön moschuskömerhaft.
Kugelrohrdestillation: Sdp.: 232°C, 0,4 mbar.
Die Angaben gelten für das Überschussisomer
¹H-NMR (200 MHz, CDCl₃): δ (ppm) = 1,16-1,36 (m, 12H), 1,42 (s, 3H), 1,56-1,60 (m, 4H), 1,87-2,19 (m, 4H), 2,28-2,39 (m, 2H), 4,00-4,18 (m, 2H), 5,04-5,26 (m, 1H).
¹³C-NMR (50MHz, CDl₃): δ (ppm) = 23,1, 24,5, 25,8, 26,2, 26,4, 26,6, 27,0, 27,2, 27,7, 34,1, 34,2, 37,9, 64,7, 121,3, 133,9, 174,2.

### Beispiel 3:

Das vorliegende Parfümöl dient zur Parfümierung vielerlei kosmetischer Produkte.

### Zusammensetzung:

| Ingredienzien | Gewichtsteile |
|---|---|
| 1. Citrophoral Base (H&R) | 5,0 |
| 2. Aldehyd C10 10 % in BA | 5,0 |
| 3. Aldehyd C 11 MOA 10 % in BA | 3,0 |
| 4. Farenal (H&R) | 3,0 |
| 5. Aldehyd C 11 10 % in IPM | 5,0 |
| 6. Citroxal 50 % in DEP | 2,0 |
| 7. trans Hex-2-enol 10 % in BA | 2,0 |
| 8. Vertocitral (H&R) | 1,0 |
| 9. Linalylacetat | 45,0 |
| 10. Citrylal (H&R) | 5,0 |
| 11. Mandarinal (Firmenich) | 4,0 |
| 12. Lilial (Givaudan Roure) | 75,0 |
| 13. Lyral (IFF) | 75,0 |
| 14. Profarnesol (H&R) | 5,0 |
| 15. Nerolidol | 5,0 |
| 16. Linalool | 45,0 |
| 17. Geraniumöl afrikanisch | 5,0 |
| 18. Phenylethylalkohol | 75,0 |
| 19. Geraniol | 15,0 |
| 20. Nerol | 10,0 |
| 21. Hexylzimtaldehyd alpha | 50,0 |
| 22. Methyldihydrojasmonat | 15,0 |
| 23. Benzylsalicylat | 100,0 |
| 24. trans,cis-2-Nonadienol 0,1 % in IPM | 5,0 |
| 25. Allylionon (Givaudan Roure) | 3,0 |
| 26. Isomethylionon gamma | 75,0 |
| 27. Eugenol | 7,0 |
| 28. Cedrylacetat | 40,0 |
| 29. Sandolen (H&R) | 5,0 |
| 30. Citral | 5,0 |

BA = Benzylalkohol; IPM = Isopropylmyristat; DEP = Diethylphtalat

Der Zusatz von
a) 355 Gewichtsteilen 5-Methyl-(E/Z)-4/(E/Z)-5-cyclopentadecenon (Summe 1000 Gewichtsteile) führt zu einer deutlich wahrnehmbaren Harmonisierung der frischen Kopfnote mit der rosig-blumigen Herznote. Darüber hinaus werden mit 5-Methyl-(E/Z)-4/(E/Z)-5-cyclopentadecenon an Moschuskömer-öl erinnernde Effekte erzielt und die feine erogene Moschusnote verleiht der vorliegenden Komposition eine hervorragende Strahlung und gesteigerte Haftung. Hierbei setzt sich besonders der wertvolle Charakter 5-Methyl-(E/Z)-4/(E/Z)-5-cyclopentadecenon im Vergleich zu Kompositionen mit konventionellen Moschusriechstoffen durch.
b) 55 Gewichtsteilen 13-Methyloxacyclohexadec-(E/Z)-12/(E/Z)-13-en-2-on (Summe 700 Gewichtsteile) verleiht der Komposition eine animalische, moschuskörnerhafte Note, die mit existierenden Moschusriechstoffen nicht erreicht wird. Weiterhin gewinnt die gesamte Komposition an Fülle und erscheint wertvoller.

## Patentansprüche

1. Verfahren zur Herstellung von Verbindungen der Formel (I) 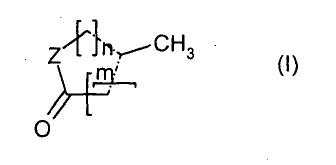 worin
Z = -CH₂- ist,
n, m ≥ 1 und n + m = 11 bis 15, bevorzugt 12 bis 14 sind, besonders bevorzugt sind n = 2, 3, 5 oder 6 und n + m = 12 bis 14,
oder
Z = O ist,
n ≥ 0, m ≥ 1 und n + m = 11 bis 15, bevorzugt 12 bis 14 sind, besonders bevorzugt sind n = 2, 3 oder 4 und n + m = 12 bis 14,
wobei die unterbrochenen Linien eine C-C-Einfach- und die andere eine C=C-Doppelbindung bedeuten,
das **dadurch gekennzeichnet ist, dass** als Edukt ein Ketolacton oder Diketon der Formel (II) 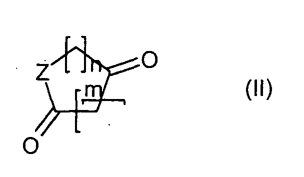 worin
Z, n und m die oben genannte Bedeutung haben,
in einer Grignard Reaktion mit Methylmagnesiumhalogenid und anschließender saurer Dehydratisierung zu den ungesättigten, methylsubstituierten makrocyclischen Ketonen und Lactonen der Formel (I) umgesetzt wird.

2. 5-Methyl-(E/Z)-4/(E/Z)-5-cyclopentadecenon
4-Methyl-(E/Z)-3/(E/Z)-4-cyclohexadecenon
6-Methyl-(E/Z)-5/(E/Z)-6-cyclohexadecenon
3-Methyl-(E/Z)-2/(E/Z)-3-cycloheptadecenon
9-Methyl-(E/Z)-8/(E/Z)-9-cycloheptadecenon
4-Methyloxacyclopentadec-(E/Z)-3/(E/Z)-4-en-2-on
5-Methyloxacyclopentadec-(E/Z)-4/(E/Z)-5-en-2-on
6-Methyloxacyclopentadec-(E/Z)-5/(E/Z)-6-en-2-on
13-Methyloxacyclopentadec-(E/Z)-12/(E/Z)-13-en-2-on
4-Methyloxacyclohexadec-(E/Z)-3/(E/Z)-4-en-2-on
5-Methyloxacyclohexadec-(E/Z)-4/(E/Z)-5-en-2-on
13-Methyloxacyclohexadec-(E/Z)-12/(E/Z)-13-en-2-on
5-Methyloxacycloheptadec-(E/Z)-4/(E/Z)-5-en-2-on
13-Methyloxacycloheptadec-(E/Z)-12/(E/Z)-13-en-2-on

3. Verbindungen der Formel (IV) 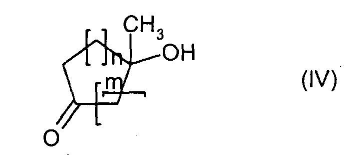 worin
n, m ≥ 1 und n + m = 11 bis 15, bevorzugt 12 bis 14 sind, besonders bevorzugt sind n = 2, 3, 5 oder 6 und n + m = 12 bis 14,
mit der Ausnahme n = 1 und m = 11.

4. Verbindungen der Formel (VI) 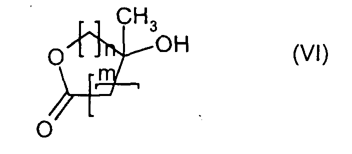 worin,
n ≥ 0, m ≥ 1 und n + m = 11 bis 15, bevorzugt 12 bis 14 sind, besonders bevorzugt sind n = 2, 3 oder 4 und n + m = 12 bis 14.

5. Verfahren zur Herstellung von Verbindungen nach einem der Ansprüche 3 bis 4, das **dadurch gekennzeichnet ist, dass** ein Diketon oder Ketolacton der Formel (II) in einer Grignard Reaktion mit Methylmagnesiumhalogenid umgesetzt wird.

6. Verwendung von Verbindungen nach dem Anspruch 2 als Riechstoffe.

7. Riechstoffmischungen enthaltend Verbindungen nach dem Anspruch 2.

8. Parfümierte Produkte enthaltend Verbindungen nach dem Anspruch 2.
